# EUROPEAN PATENT APPLICATION

(11) **EP 4 467 006 A1**
(43) Date of publication of application: **27.11.2024**
(21) Application number: 23174488.9
(22) Date of filing: 22.05.2023
(51) Int. Cl.: A23L 33/00, A61K 9/00, A61K 31/194, A61K 31/197, A61K 36/258, A61P 25/26

(54) **COMPOSITION FOR PREVENTION AND/OR TREATMENT OF MENTAL AND PHYSICAL EXHAUSTION CAUSED BY CIRCADIAN RHYTHM SLEEP DISORDER**

(71) Applicant: Bobok, Maxim, Moscow 140013 (RU)
(72) Inventor: Bobok, Maxim, Moscow 140013 (RU)
(74) Representative: Pribic, Jelena

(57) **Abstract**

The invention relates to a composition for prevention of mental and physical exhaustion preferably caused by sleep-wake rhythm disturbances. The composition contains ginseng roots extract, γ-Aminobutyric acid (GABA), succinic acid, and excipients. The present composition may be in the form of hard gelatin capsules with granulate, effervescent tablets or gummies. The composition according to the invention is demonstrated to provide a reduction of fatigue caused by circadian rhythm sleep disorder and increases physical and mental performance of the subject.

## Description

### Technical Field

The present invention relates to a composition for prevention and/or treatment of mental and physical exhaustion conditions. Particularly, the claimed composition may be used for prevention and/or treatment of mental and physical exhaustion conditions caused by sleep-wake rhythm disturbances. Moreover, this invention relates to a method for preparation of said composition.

### Background Art

Circadian rhythm is a biological rhythm based on a 24/hour cycle of biological processes. Different physiological processes in body are driven by the circadian rhythm. In addition to sleep, this rhythm regulates body temperature, heart activity, hormone secretion, blood pressure, oxygen consumption, metabolism, and many other functions. Exhaustion due to circadian rhythm sleep disorder and, as a result, disruption of the adaptive mechanisms have significant impact on the regulation of homeostasis of living organisms. Namely, violation of the natural structure of biorhythms negatively affects the processes of restoration of organs and systems, leading to their overstrain and premature depletion of biological resources.

If the normal periods and phases between circadian rhythms of the body and environment are disturbed, a stress can be developed, further resulting in mental and/or physical exhaustion (rapid fatigue, general malaise, daytime sleepiness, difficulty in sleeping at night, and reduced physical and mental performance). Deterioration of physical and mental performance generally causes a decrease in the well-being and impairment of the quality of life. The circadian rhythm sleep disorder can further manifest through somatic symptoms mainly associated with gastrointestinal dysfunction, muscle pain or headache [Roach, G. D., & Sargent, C. (2019). Interventions to Minimize Jet Lag After Westward and Eastward Flight. Frontiers in Physiology]. People who often suffer from circadian rhythm sleep disorder (pilots, diplomats, politicians, heads of multinational corporations, athletes, shift workers, residents of large cities, office workers) are at high risk of developing various diseases, particularly chronic diseases (primarily of the CNS, cardiovascular, and endocrine system), including neoplastic diseases.

Among the prior art approaches that improve a person's adaptation to changing living conditions, there are non-pharmaceutical (light therapy, adherence to sleep-wake regime, a healthy sleep environment, being outdoors, cold water treatment) and pharmaceutical methods of treatment. Well known regulator of the circadian rhythm of the body is melatonin, as well as metabolites thereof. The prior art product on the global market is Melaxen^{®}, with melatonin as an active substance. Melatonin is involved in the formation of daily circadian rhythms, inhibition of certain functions of the pituitary gland, regulation of immune responses, has analgesic and sedative effects [Tordjman, S., Chokron, S., Delorme, R., Charrier, A., Bellissant, E., Jaafari, N., & Fougerou, C. (2017). Melatonin: Pharmacology, Functions and Therapeutic Benefits. Current Neuropharmacology, 15(3), 434-443]. However, certain cases of development of adverse effects during the period of melatonin therapy, including daytime sleepiness, headache, dizziness, hypothermia, agitation, fatigue, mood swings, nightmares, skin irritation and palpitations [Besag FMC, Vasey MJ, Lao KSJ, Wong ICK. Adverse Events Associated with Melatonin for the Treatment of Primary or Secondary Sleep Disorders are reported: A Systematic Review. CNS Drugs 2019; 33(12):1167-1186]. Moreover, there are several contraindications for administration of Melaxen^{®}: severe renal dysfunction, autoimmune diseases, leukemia, lymphoma, allergic reactions, lymphogranulomatosis, myeloma, epilepsy, pregnancy and breastfeeding [Savage RA, Zafar N, Yohannan S, et al. Melatonin. [Updated 2022]. In: StatPearls [Internet]. Treasure Island (FL): StatPearls Publishing; 2022. Available from: https://www.ncbi.nlm.nih.gov/books/NBK534823/].

As an alternative, herbal adaptogens can be used for the relief of symptoms of asthenia (abnormal loss of strength and energy) such as weakness caused by circadian rhythm sleep disorder [RU Patent No. 2735833C1; RU Patent No. 2674337C1, RU Patent No.2493866C2, WO 2006057578A2. Adaptogens have neuroprotective and tonic properties on CNS, antioxidant, hepatoprotective, antitumor and immunomodulatory activity [Panossian, A.; Efferth, T. Network Pharmacology of Adaptogens in the Assessment of Their Pleiotropic Therapeutic Activity. Pharmaceuticals 2022, 15, 1051].

Patent document WO2016204646A1 discloses a composition for prevention of mental and physical exhaustion caused by circadian rhythm sleep disorder. The disclosed composition contains extract of Eleutherococcus, succinic acid and pyridoxine hydrochloride (vitamin B6). In another study, the use of combined multivitamin supplements, as well as individual vitamins, including the B complex, was shown to impair sleep maintenance and cause higher rates of insomnia [Syed EU, Wasay M, Awan S. Vitamin B12 Supplementation in Treating Major Depressive Disorder: A Randomized Controlled Trial. Open Neurol J. 2013; 7:44-48].

Document RU 2577701C1 describes a composition consisting of a mixture of plant extracts containing dry extract of Siberian Ginseng (Eleutherococcus senticosus) and dry yerba mate (Ilex paraguariensis). The composition displays a chronocorrective effect and adaptogenic activity when light desynchronosis is exposed.

### Technical Problem

Nowadays, human life is characterized by high level of urbanization, which consequently leads to hypodynamia and psycho-emotional stress. Additionally, the problem of reducing physical performance is highly relevant for healthy people. Circadian rhythm sleep disorders are caused by continuous or occasional disruption of sleep patterns, i.e. inconsistency between sleep-wake rhythm and external rhythm of the change of day and night. Particularly, this debilitating condition can be attributed to a misalignment between the body's internal clock and the external environment (for example, social and work schedules). Therefore, there is a need for development of an effective composition for prevention and/or treatment of mental and physical exhaustion, particularly caused by circadian rhythm sleep disorder, which has an improved safety profile with significantly decreased number of side effects observed in the compositions known so far.

### Summary of the invention

The above-mentioned drawbacks of the prior art have been solved by a composition according to the present invention, the composition comprising a dry extract of ginseng roots (Panax ginseng C. A. Mey) of the Araliaceae family, a γ-Aminobutyric acid (GABA) and a succinic acid with one or more pharmaceutically acceptable excipients. The present composition may be used as an agent for the prevention and/or treatment of mental and physical exhaustion caused by circadian rhythm sleep disorder. Administration of the present composition promotes the central defense systems of the body. Namely, having an adaptogenic and anxiolytic effect, the composition according to the present invention improves the central nervous and endocrine systems and exhibits safety and efficacy in leveling the effects of sleep-wake rhythm disturbances.

Administration of the present composition results in decrease of the fatigue, normalizes the sleep-wake rhythm, increases the physical and mental performance, while at the same time causes significantly reduced number of side effects. Advantageously, this composition does not irritate the gastric mucosa. The claimed composition is intended for oral administration in the form of hard gelatin capsules with granulate, effervescent tablets, gummies or any other form suitable for *per os* application.

### Detailed description of the invention

Exemplary composition according to the present invention is intended for use for prevention of mental and physical exhaustion caused by circadian rhythm sleep disorder. The composition comprises: dry extract of ginseng roots ranging from 18.5-30.9% by weight, γ-Aminobutyric acid ranging from 30.9-60.7% by weight, succinic acid ranging from 9.6-18.5% by weight, excipients ranging from 15.8-30.6% by weight. Suitable excipients to be used in the present composition may be selected from potato and corn starch, lactose, sucrose, maltose, microcrystalline cellulose, gelatin, agave syrup, sodium starch glycolate, croscarmellose sodium, cyclodextrins, malic acid, tartaric acid, polyvinylpyrrolidone, mannitol, colloidal silicon dioxide, sodium bicarbonate, magnesium stearate, stearic acid, citric acid, water, polyvinylpyrrolidone (PVP), polyethylene glycol (PEG) or combination thereof. The composition according to the present invention diminishes the number of side effects, thus providing improved safety when compared with the compositions already known.

Panax ginseng, or ginseng (Panax ginseng C. A. Mey) is a perennial herbaceous plant of the genus Ginseng from the Araliaceae family. The roots of the plant are used as raw materials for obtaining a liquid and dry extract. Panax ginseng is one of the most widely used adaptogens used for increasing nonspecific resistance of the organism to extreme conditions. A comprehensive effect on the body results from the presence of the following physiologically active substances in the root: panaxin substance which tones the heart and blood vessels; panaxic acid which actively affects the metabolism, enhancing oxidative processes leads to the rapid breakdown of fats; panaquilon which stimulates the endocrine system and helps formation of the necessary level of hormones in the body; panaxen essential oil which has an analgesic and calming effect on CNS; ginsenin glycoside which lowers blood sugar [Jin, T., Rong, P., Liang, H., Zhang, P., Zheng, G., & Lin, Y. (2020). Clinical and Preclinical Systematic Review of Panax ginseng C. A. Mey and Its Compounds for Fatigue. Frontiers in Pharmacology]. The positive effect of the ginseng active compounds on well night sleep has been well known in the art.

GABA is an inhibitory neurotransmitter found primarily in the human brain and eyes. It regulates many inhibitory and sedative processes occurring in the brain tissue; therefore, it is extremely important for relaxation. GABA activates the energy processes, restores metabolic processes, promotes the utilization of glucose, remove of toxic metabolic products, ensures the normalization of the dynamics of nervous processes in the brain, increases the productivity of thinking and improves memory.

The use of succinic acid and its salts helps neutralization of circadian rhythm sleep disorder effect and pathological adaptive reactions by reducing the process of lipid peroxidation [WO2010062206A1]. Conversion of succinic acid in the body, mainly through the oxidation thereof, is associated with the production of energy necessary for life, particularly in conditions of increasing load on any of the body's systems. It is important to note that the power of the succinic acid-based energy production system is significantly greater than all other energy formations of the body [Ives, S. J., Zaleski, K. S., Slocum, C., Escudero, D., Sheridan, C., Legesse, S., Vidal, K., Lagalwar, S., & Reynolds, T. H. (2020). The effect of succinic acid on the metabolic profile in high-fat diet-induced obesity and insulin resistance. Physiological Reports, 8(21)].

### Experiments

Series of experiments has been conducted revealing the effectiveness of the composition according to present application when used in prevention and/or treatment of mental and physical exhaustion caused by circadian rhythm sleep disorder.

In one embodiment of the present invention a mixture of dry extract of ginseng roots, γ-Aminobutyric acid and Succinic acid is combined with Lactose, Microcrystalline cellulose and Magnesium stearate.

In second embodiment, the mixture of dry extract of ginseng roots, γ-Aminobutyric acid and Succinic acid is combined with gelatin, agave syrup and maltose syrup.

In third embodiment, the mixture of dry extract of ginseng roots, γ-Aminobutyric acid and Succinic acid is combined with sodium bicarbonate, citric acid, sucrose and magnesium stearate.

### Examples

### Example I

Dry extract of ginseng roots - 400 mg:
γ-Aminebulyric acid - 750 mg;
Succinic acid - 200 mg;
Lactose - 1.60 mg;
Microcrystalline cellulose - 100 mg;
Magnesium stearate - 10 mg.

### Example 2

Dry extract of ginseng roots - 300 mg;
γ-Aminobutyric acid - 500 mg;
Succinic acid - 150 mg;
C;c-latin - 475 mg;
Agave syrup - 475 mg;
Maltose syrup - 3800 mg.

### Example 3

Dry extract of ginseng roots - 400 mg;
γ-Aminobutyric acid - 1000 mg;
Succinic acid - 200 mg;
Sodium bicarbonate - 2000 mg:
Citric acid - 800 mg;
Sucrose - 400 mg:
Magnesium stearate - 50 mg,

The study of the pharmacological activity of a composition (GGS) comprising a dry extract of Ginseng roots, GABA and Succinic acid (GGS) on the model of "light desynchronosis" was performed. Four groups of experimental animals (white outbred mice and Wistar rats) have been formed, each group consisting of 16 animals, and each animal was administered orally with GGS composition or with a control. Group-I was not exposed to the light and received distilled water. Group-I was used as a positive control. Group-II was for 14 days constantly exposed to the light and received distilled water. Group-II was used as a negative control. Group III received Melaxen^{®} and Group-IV received daily dose of GGS composition in animal equivalent doses (AED). Thus, Groups II, III and IV had 14 days of constant light exposure [M.N. Bobok, L.A. Pavlova, S.V. Kozin, The Effect of light desynchronosis on the duration of forced swimming in mice // Biomedicine. 2017. No. 1]. Standard assays, known in the art, were used to evaluate the effect of the composition administered: 1) duration of swimming of laboratory animals according to the forced swim test (FST); 2) changing the masses of stress-competent organs (liver, spleen, thymus, adrenal glands); 3) assessing of influence on orienting-exploratory behavior of rats in the tests Open field (OF) and Elevated plus maze (EPM); 4) assessing tonic activity on a thiopental sodium induced sleeping time test.

Administration of GGS in the dose lower than 300/500/100 mg of dry extract of ginseng roots, GABA, succinic acid, respectively, did not show significant increase in the duration of swimming animals compared with the control groups. Also, the mass of organs of the animals that were subjected to the stress differed insignificantly from the mass of organs of animals that did not receive the test agent.

With the administration of GGS in the dose higher than 500/1000/300 mg of dry extract of ginseng roots, GABA, succinic acid, respectively, swimming duration increased by 10% compared with the results of the positive control group. The mass of organs of the animals that were subjected to the stress also differed insignificantly from the mass of organs of positive and negative control groups. In addition, with administration of high doses, an irritating effect of succinic acid on the wall of the gastric mucosa was observed.

The best results in mise models were obtained with the GGS composition comprising dry extract of ginseng roots in the dose range from 300-500 mg, GABA in the dose range from 500-1000 mg, and succinic acid in the dose range from 100-300 mg. Table 1 represents the specific organ mass-change (wt%) and results from the force-swimming test for tested animals. It was shown that the GGS treatment group had the swimming effectiveness increased by 41% compared to the negative control group and 12% compared to the positive control group. It was found that the mass of stress-competent organs in the GSS treatment group differed insignificantly from the mass of organs of animals that did not have light desynchronosis (positive control).

**Table 1: Changes in the mass of specific stress-responsive organs and FST (M±m) after simulated light desynchronosis**

| Groups | Organs (wt%) | | | | FST (%) |
|---|---|---|---|---|---|
| | Liver | Spleen | Thymus | Adrenals | |
| Positive control | 5.41±0.28 | 0.43±0.02 | 0.31±0.03 | 0.13±0.02 | 553,1±32,9^{∗} |
| Negative control | 6.81±0.23 | 0.38±0.02 | 0.27±0.03 | 0.19±0.02 | 438,4±37,3^{∗∗} |
| Melaxen^{®} | 5.85±0.18 | 0.42±0.04 | 0.28±0.02 | 0.14±0.02 | 471,1±41,4^{∗},^{∗∗} |
| GGS | 5.26±0.28 | 0.4±0.03 | 0.31±0.02 | 0.12±0.02 | **621,8±36,8**^{∗},^{∗∗} |

In the EPM test, after the administration of GGS, rats did not experience anxiety and fear of the novelty of the situation. Thus, after administration of the GGS composition, the anxiety index was significantly lower compared with the same indicator of the negative control (0.68 vs 0.84). The use of GGS composition at a daily dose contributed to an increase in the number of visits to the open arm by 3 times compared with the negative control group. Moreover, the time spent in open arms increased compared to the negative control group by 2.8. Total distance and time in center of the GGS group were significantly higher compared with the same indicator of the negative control (1.46 and 2.2 times respectively) in open field test, which indicates an anxiolytic effect of the GGS composition. Results of EPM and OF tests are summarized in Table 2 below.

**Table 2: Orienting and exploratory behaviors in EPM and OF (M ± m) after simulated light desynchronosis**

| Groups | EPM | | | | OF | |
|---|---|---|---|---|---|---|
| | Open arms (sec) | Closed arms (sec) | Center (sec) | Anxiety index | Distance traveled (cm) | Time in center areas (sec) |
| Positive control | 52.5±9.7 | 201.0±15.5 | 21.3±2.1 | 0.75 | 1506.25±215.2 | 131.17±35.15 |
| Negative control | 23.75±9.6 | 245.0±13.9 | 14.9±2.6 | 0.84 | 908.21±216.13 | 69.98 ±29.37 |
| Malaxen^{®} | 44.2±8.4 | 224.2±20.4 | 14.24±3.1 | 0.76 | 1421.65±228.28 | 119.62±23.42 |
| GGS | 65.0±11.5 | 203.5±13.3 | 21.45±2.9 | **0.68** | 1332.48±197.43 | 154.19±36.95 |

The obtained results in thiopental sodium induced sleeping time test indicate a pronounced antihypnogenic effect of GGS composition (Table 3). In the negative control group onset of action and duration of sleeping were longer compared to all other groups. Against the background of the action of GGS composition, compared with the negative control, the duration of thiopental sleep was similar to the positive control group.

**Table 3: Parameters of thiopental sodium induced sleeping time test**

| Groups | Onset of action (min) | Duration of sleeping (min) | Effect (%) |
|---|---|---|---|
| Positive control | 6.2±1.2 | 58.8±4.9 | 0 |
| Negative control | 16.5±2.2 | 27.6±3.8 | 46.94 |
| Malaxen^{®} | 8.4±1.3 | 49.1±1.6 | 83.50 |
| GGS | 7.3±1.5 | 54.9±3.6 | **93.37** |

Hence, the results of the study indicate an improvement in physical endurance, orienting-exploratory behavior, tonic activity of the experimental animals received the claimed composition. According to the study, during which the effectiveness of the administrated GGS dose was examined, a positive effect on stress-competent organs with a simulated physical exhaustion caused by circadian rhythm sleep disorder is demonstrated.

The oral composition according to the invention may be in the form of granulate in sachets, solution, suspension, capsule, hard gelatin capsules with granules, effervescent tablets, gummies or similar.

The process for obtaining the composition according to the present invention includes the following steps:
preparation of a dry extract of ginseng roots using well-known methods for preparation dry extracts,
addition of γ-aminobutyric acid and succinic acid and
addition of the suitable excipient(s) and obtaining a mixture.

The composition according to the present invention may be used as a medicine or a dietary supplement.

The daily dose of the composition must contain:
dry extract of ginseng roots in the dose range from 300-500 mg, preferably 400 mg
GABA in the dose range from 500-1000 mg, preferably 750 mg
and succinic acid in the dose range from 100-300 mg, preferably 200 mg.

The composition according to the present invention provides a reduction of fatigue caused by circadian rhythm sleep disorder and increase in physical and mental performance of the subject.

## Claims

1. A composition for prevention and/or treatment of mental and physical exhaustion, comprising:
ginseng roots extract,
γ-aminobutyric acid and
succinic acid.

2. The composition according to claim 1 comprising:
ginseng roots extract 18.5-30.9 wt%,
γ-aminobutyric acid 30.9-60.7 wt%,
succinic acid 9.6-18.5 wt%.

3. The composition according to claim 1 or 2 further comprising an excipient.

4. The composition according to claim 3 comprising the excipient 15.8-30.6 wt%.

5. The composition according to claim 3 or 4, wherein the excipient is selected from the group containing: potato and corn starch, sodium starch glycolate, croscarmellose sodium, cyclodextrins, malic acid, tartaric acid, polyvinylpyrrolidone, mannitol, colloidal silicon dioxide, stearic acid or combination thereof.

6. A product comprising the composition according to claims 1 to 5 for use as a medicament or a dietary supplement.

7. The product according to claim 6 for use in the treatment of mental and physical exhaustion caused by circadian rhythm sleep disorder.

8. The product for prevention and/or treatment of mental and physical exhaustion, wherein the product comprises:
ginseng roots extract,
γ-aminobutyric acid,
succinic acid.

9. The product according to claim 8, wherein the product comprises:
ginseng roots extract 300-500 mg,
γ-aminobutyric acid 500-1000 mg,
succinic acid 100-300 mg.

10. The product according to claim 8, wherein the product comprises:
ginseng roots extract 400 mg,
γ-aminobutyric acid 750 mg,
succinic acid 200 mg,
lactose 160 mg,
microcrystalline cellulose 100 mg,
magnesium stearate 10 mg.

11. The product according to claims claim 8, wherein the product comprises:
ginseng roots extract 300 mg,
γ-aminobutyric acid 500 mg,
succinic acid 150 mg
gelatin 475 mg,
agave syrup 475 mg,
maltose syrup 3800 mg,

12. The product according to claims claim 8, wherein the product comprises:
ginseng roots extract 1000 mg,
γ-aminobutyric acid 500 mg,
succinic acid 200 mg
sodium bicarbonate 2000 mg,
citric acid 800 mg,
sucrose 400 mg,
magnesium stearate 50 mg.

13. The product according to claims 6 to 12, wherein the product is in the form of coated granules in a sachet, a solution, a suspension, a capsule, a tablet, an effervescent tablet or a gummy.

14. The product according to claim 13, wherein the capsule is a hard gelatin capsule with granulate.

15. A method of preparing the composition according to claims 1 to 5, wherein the method includes the following steps:
preparation of an extract of ginseng roots,
addition of γ-aminobutyric acid and succinic acid and
addition of the suitable excipient(s) and obtaining a mixture.
